# EUROPÄISCHE PATENTANMELDUNG

(11) **EP 3 620 209 A1**
(43) Veröffentlichungstag der Anmeldung: **11.03.2020**
(21) Anmeldenummer: 18192360.8
(22) Anmeldetag: 04.09.2018
(51) Int. Cl.: A61N 1/37, A61B 5/00, A61B 5/042, A61N 1/39

(54) **PASSIVER SENSOR ZUR DRAHTLOSEN DETEKTION DER ELEKTRISCHEN ERREGUNG VON MUSKELZELLEN**

(71) Anmelder: BIOTRONIK SE & Co. KG, 12359 Berlin (DE)
(72) Erfinder: RUMP, Jens, 12049 Berlin (DE); MOSS, Christian, 10589 Berlin (DE)
(74) Vertreter: Engelhardt, Björn

(57) **Zusammenfassung**

Die Offenbarung betrifft einen implantierbarer Sensor (1) zur Detektion einer elektrischen Erregung von Muskelzellen (M), insbesondere Herzmuskelzellen (M), wobei vorgesehen ist, dass der Sensor (1) eine dielektrische Komponente (2) sowie eine Kontaktstelle (20) zum Kontaktieren von Muskelzellen (M) aufweist, die mit der dielektrischen Komponente (2) verbunden ist, so dass sich bei einer elektrischen Erregung der Muskelzellen (M) ein elektrisches Feld in der dielektrischen Komponente (2) und entsprechend eine Kapazität der dielektrischen Komponente (2) ändert. Des Weiteren ist ein System mit einem Sensor und einem Implantat offenbart.

## Beschreibung

Die vorliegende Offenbarung betrifft einen implantierbaren Sensor zur Detektion einer elektrischen Erregung von Muskelzellen, insbesondere Herzmuskelzellen.

### Hintergrund

Es ist bekannt, die elektrische Erregung von Muskelzellen mit Hilfe von kabelgebundenen Elektroden zu detektieren.

Aufgrund der damit verbundenen Probleme wie erhöhter Infektionsgefahr oder Kabelbruch, gibt es bereits Bemühungen, kabellose Lösungen als Ersatz zu entwickeln. Eine Herausforderung stellt die Notwendigkeit dar, dass bei einigen Therapieformen, insbesondere bei der Therapie von Tachykardien, zusätzlich zur aktiven Therapie ein Sensor im Atrium, üblicherweise in unmittelbarer Nähe zum Koronarsinusknoten, platziert werden muss, welcher die Herzaktivität überwacht.

Die Signalübertragung vom Sensor zum aktiven Implantat wird dabei durch eine Kabelverbindung realisiert. Ein alternatives Konzept ist es, dass der Sensor über eine eigene Stromversorgung verfügt und die Signale drahtlos an das Implantat übermittelt.

Bei einer aktiven Stromversorgung mittels einer Batterie besteht die Problematik eines größeren Formfaktors sowie einer begrenzten Lebensdauer. Der Sensor ist entsprechend aufwendiger hinsichtlich seines Designs.

Weiterhin ist bei kabelgebundenen Elektroden eine galvanische Verbindung nötig, die anfällig gegenüber mechanischen Fehlern ist.

### Zusammenfassung

Hiervon ausgehend liegt der vorliegenden Erfindung die Aufgabe zugrunde, einen Sensor bereitzustellen, der hinsichtlich der vorgenannten Problematik verbessert ist.

Diese Aufgabe wird durch einen implantierbaren Sensor mit den Merkmalen des Anspruchs 1 gelöst. Weitere Ausführungsformen sind in den Unteransprüchen angegeben und werden nachfolgend beschrieben.

Gemäß Anspruch 1 wird ein implantierbarer Sensor zur Detektion einer elektrischen Erregung von Muskelzellen, insbesondere Herzmuskelzellen, offenbart, wobei sich der Sensor dadurch auszeichnet, dass er eine dielektrische Komponente sowie eine Kontaktstelle zum elektrischen Kontaktieren von Muskelzellen eines Patienten aufweist, die elektrisch leitend mit der dielektrischen Komponente verbunden ist, so dass sich bei einer elektrischen Erregung der Muskelzellen ein elektrisches Feld in der dielektrischen Komponente und entsprechend eine Kapazität der dielektrischen Komponente ändert.

Die Erfindung ermöglicht somit mit Vorteil eine passive Messung der Reizüberleitung von Herzmuskelzellen, insbesondere zur weiteren Signalverarbeitung in einem aktiven Implantat, basierend auf der Änderung des elektrischen Feldes bzw. der Kapazität der dielektrischen Komponente, und zwar insbesondere ohne eine autonome Energieversorgung des Sensors.

Gemäß einer Ausführungsform ist vorgesehen, dass der Sensor ein passiver Sensor ist.

Weiterhin ist gemäß einer Ausführungsform vorgesehen, dass der Sensor zur drahtlosen Übertragung der Detektion einer Erregung der Muskelzellen auf ein medizinisches Gerät, insbesondere ein implantierbares medizinisches Gerät, ausgebildet ist.

Weiterhin ist gemäß einer Ausführungsform vorgesehen, dass der Sensor eine Antennenstruktur aufweist, die mit der dielektrischen Komponente verbunden ist.

Gemäß einer Ausführungsform ist diesbezüglich vorgesehen, dass die Antennenstruktur so mit der dielektrischen Komponente verbunden ist, dass sich die Anpassung der Antennenstruktur verändert, wenn sich bei einer elektrischen Erregung der Muskelzellen das elektrische Feld in der dielektrischen Komponente und die Kapazität der dielektrischen Komponente ändert.

Dies kann mit Hilfe eines breitbandigen elektromagnetischen Signals, welches vom Implantat zum Sensor gesendet wird, gemessen werden. Grundsätzlich kommen dafür insbesondere alle für die drahtlose Kommunikation zugelassenen Frequenzen in Frage, wobei jedoch bei steigender Frequenz die Reichweite abnimmt. Demgegenüber nimmt die Sensitivität mit der Frequenz zu. Entsprechend können bspw. Frequenzen im Bereich von 1 kHz (rein induktive Kopplung) bis 1 GHz (elektromagnetische Kopplung), insbesondere im Bereich von 13 MHz (hauptsächlich induktive Kopplung), verwendet werden.

Somit kann ein weiteres (insbesondere implantierbares) medizinisches Gerät (wie z. B. ein ICD oder ein Herzschrittmacher) eine elektrische Erregung der Muskelzellen auf dem Wege einer drahtlosen Kommunikation erfahren bzw. vom Sensor abfragen bzw. mit Hilfe des Sensors messen.

Gemäß einer Ausführungsform ist vorgesehen, dass die Antennenstruktur einen elektrischen Leiter aufweist, der ein erstes Ende und ein zweites Ende aufweist, wobei die beiden Enden jeweils mit der dielektrischen Komponente verbunden sind.

Weiterhin ist gemäß einer Ausführungsform vorgesehen, dass der besagte elektrische Leiter schlaufenförmig ausgebildet ist.

Weiterhin kann die Antennenstruktur gemäß einer Ausführungsform einen weiteren elektrischen Leiter aufweisen, der vorzugsweise ebenfalls schlaufenförmig ausgebildet sein kann, wobei der weitere elektrische Leiter ein erstes Ende und ein zweites Ende aufweist, wobei die beiden Enden des weiteren elektrischen Leiters wiederum jeweils mit der dielektrischen Komponente verbunden sind.

Gemäß einer hinsichtlich der Antennenstruktur alternativen Ausführungsform ist vorgesehen, dass der Sensor eine Induktivität in Form einer Spulenstruktur aufweist, die mit der dielektrischen Komponente verbunden ist. Die Spulenstruktur kann z. B. durch eine Spule gebildet sein, die einen elektrischen Leiter mit mehreren Windungen aufweist, wobei der elektrische Leiter zwei Enden aufweist, die mit der dielektrischen Komponente verbunden sind, insbesondere galvanisch, z. B. durch eine Schweißverbindung, eine Lötverbindung oder eine elektrisch leitende Klebeverbindung.

Gemäß einer Ausführungsform kann diesbezüglich vorgesehen sein, dass die Spulenstruktur und die dielektrische Komponente einen Schwingkreis bilden, so dass sich eine Resonanzfrequenz des Schwingkreises ändert, wenn sich bei einer elektrischen Erregung der Muskelzellen das elektrische Feld in der dielektrischen Komponente und die Kapazität der dielektrischen Komponente ändert.

Die Änderung der Resonanzfrequenz kann wiederum mit bekannten Verfahren (auf dem Wege einer drahtlosen Kommunikation) ermittelt werden, z. B. durch ein (insbesondere implantierbares) medizinisches Gerät (z. B. in Form eines ICD oder eines Herzschrittmachers).

Weiterhin ist gemäß einer Ausführungsform vorgesehen, dass die Spulenstruktur zwei spiralförmige elektrische Leiter aufweist, wobei die beiden spiralförmigen elektrischen Leiter jeweils mit der dielektrischen Komponente verbunden sind, wobei insbesondere der jeweilige spiralförmige elektrische Leiter über ein Ende des jeweiligen spiralförmigen Leiters mit der dielektrischen Komponente verbunden ist.

Weiterhin ist gemäß einer Ausführungsform vorgesehen, dass der Sensor ein Befestigungselement zum Befestigen des Sensors am Gewebe (insbesondere Muskelgewebe bzw. Herzmuskelgewebe) eines Patienten aufweist, wobei das Befestigungselement insbesondere an der dielektrischen Komponente festgelegt ist.

Gemäß einer Ausführungsform kann das Befestigungselement schraubenförmig ausgebildet sein (z. B. in Form eines helixförmigen Drahts).

Weiterhin ist gemäß einer Ausführungsform vorgesehen, dass die Kontaktstelle durch das Befestigungselement gebildet ist.

Weiterhin ist gemäß einer Ausführungsform vorgesehen, dass der Sensor eine weitere Kontaktstelle zum elektrischen Kontaktieren von Muskelzellen eines Patienten aufweist, die elektrisch leitend mit der dielektrischen Komponente verbunden ist.

Gemäß einer Ausführungsform des Sensors ist weiterhin vorgesehen, dass die dielektrische Komponente einen Kondensator aufweist oder durch einen Kondensator gebildet ist, wobei es sich bei dem Kondensator insbesondere um einen Keramikkondensator der Klasse 2 handelt.

Gemäß einer weiteren Ausführungsform ist vorgesehen, dass die dielektrische Komponente (bzw. der Kondensator) einen ferroelektrischen Stoff aufweist, insbesondere Bariumtitanat oder Blei-Zirkonat-Titanat.

Ein weiterer Aspekt betrifft ein System, aufweisend einen hier offenbarten implantierbaren Sensor sowie ein implantierbares medizinisches Gerät (insbesondere in Form eines implantierbaren Herzschrittmachers oder eines implantierbaren Kardioverter-Defibrillators), wobei das medizinische Gerät zum vorzugsweise drahtlosen Messen bzw. Abfragen einer vom Sensor detektierten Erregung von Muskelzellen konfiguriert ist.

Insbesondere für den Fall, dass eine Antennenstruktur verwendet wird (siehe oben), kann das implantierbare medizinische Gerät dazu ausgebildet sein, ein vorzugsweise breitbandiges elektromagnetisches Signal zu dem Sensor zu senden und das Reflexionsverhalten der Antennenstruktur auszuwerten, das ein Maß für die Aktivität der elektrischen Erregung der Muskelzellen bzw. Herzmuskelzellen darstellt, die am Ort der dielektrischen Komponente herrscht. Die Breitbandigkeit kann z. B. anhand der Kapazitätsänderung und damit der Änderung der Resonanzfrequenz des Sensors ausgewählt werden. So kann z. B. eine Bandbreite im Bereich von 10 kHz verwendet werden. Je nach verwendetem Frequenzbereich ist es vorteilhaft, wenn die Bandbreite zumindest 10% der Mittelfrequenz beträgt. Alternativ kann die Resonanzfrequenz auch mittels eines Frequenzsweeps für diesen Frequenzbereich erfolgen.

Insbesondere für den Fall, dass alternativ eine Induktivität in Form einer Spulenstruktur verwendet wird, die mit der dielektrischen Komponente einen Schwingkreis bildet (siehe oben), kann das implantierbare medizinische Gerät dazu ausgebildet sein, die Resonanzfrequenz des Schwingkreises zu messen, die sich bei einer elektrischen Erregung von Muskelzellen verändert.

Das System kann mehrere Sensoren aufweisen, um insbesondere die Messergebnisse zu verbessern. Es können dabei auch mehrere verschiedene Sensoren eingesetzt werden. Die Sensoren können dann z. B. durch das implantierbare medizinische Gerät einzeln und/oder zusammen abgefragt werden.

### Beschreibung von Ausführungsformen

Im Folgenden sollen beispielhafte Ausführungsformen anhand der Figuren detailliert beschrieben werden. Es zeigen:
- Fig. 1: eine schematische Darstellung einer Ausführungsform eines Sensors mit einer dielektrischen Komponente und einer Antennenstruktur,
- Fig. 2: eine schematische Darstellung einer weiteren Ausführungsform eines Sensors mit einer dielektrischen Komponente und einer Antennenstruktur,
- Fig. 3: eine schematische Darstellung einer weiteren Ausführungsform eines Sensors mit einer dielektrischen Komponente und einer Spulenstruktur,
- Fig. 4: eine schematische Darstellung einer weiteren Ausführungsform eines Sensors mit einer dielektrischen Komponente und einer Spulenstruktur,
- Fig. 5: zeigt einen implantierten, in einer Herzwand verankerten Sensor sowie eine Elektrodenleitung mit Antenne zum Abfragen der elektrischen Erregung der mittels des Sensors kontaktierten Muskelzellen, wobei die abfragende Antenne innerhalb des Herzens (A) oder außerhalb des Herzens (B) positionierbar ist,
- Fig. 6: zeigt die Möglichkeit der Verwendung mehrerer Sensoren zum Detektieren von elektrischen Erregungen von Muskelzellen bzw. Herzmuskelzellen,
- Fig. 7: eine schematische Darstellung eines Kondensators bzw. einer Kapazität,
- Fig. 8: eine Darstellung der Änderung der Kapazität in Abhängigkeit einer anliegenden Spannung, und
- Fig. 9: eine Darstellung der Änderung der Resonanzfrequenz in Abhängigkeit der anliegenden Spannung.

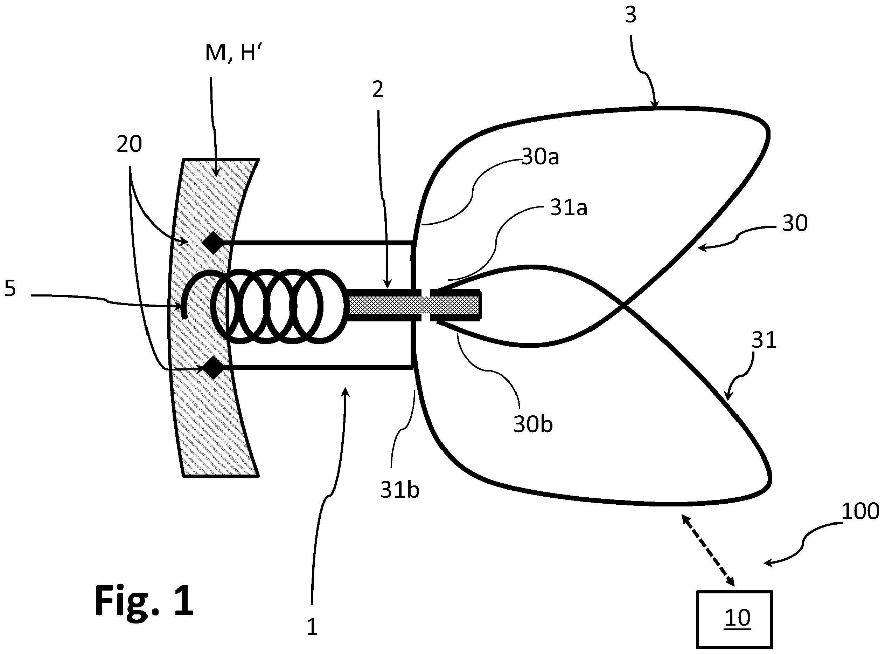

Die Offenbarung betrifft einen implantierbarer Sensor 1 zur Detektion einer elektrischen Erregung von Muskelzellen M, insbesondere Herzmuskelzellen M eines Herzens H eines Patienten, wie er z. B. in den Figuren 1 bis 4 dargestellt ist.

Dabei ist vorgesehen, dass der Sensor 1 eine dielektrische Komponente 2 sowie zumindest eine Kontaktstelle 20 zum Kontaktieren der Muskelzellen M aufweist, die mit der dielektrischen Komponente 2 verbunden ist, so dass sich bei einer elektrischen Erregung der Muskelzellen M ein elektrisches Feld in der dielektrischen Komponente 2 und entsprechend eine Kapazität der dielektrischen Komponente 2 ändert.

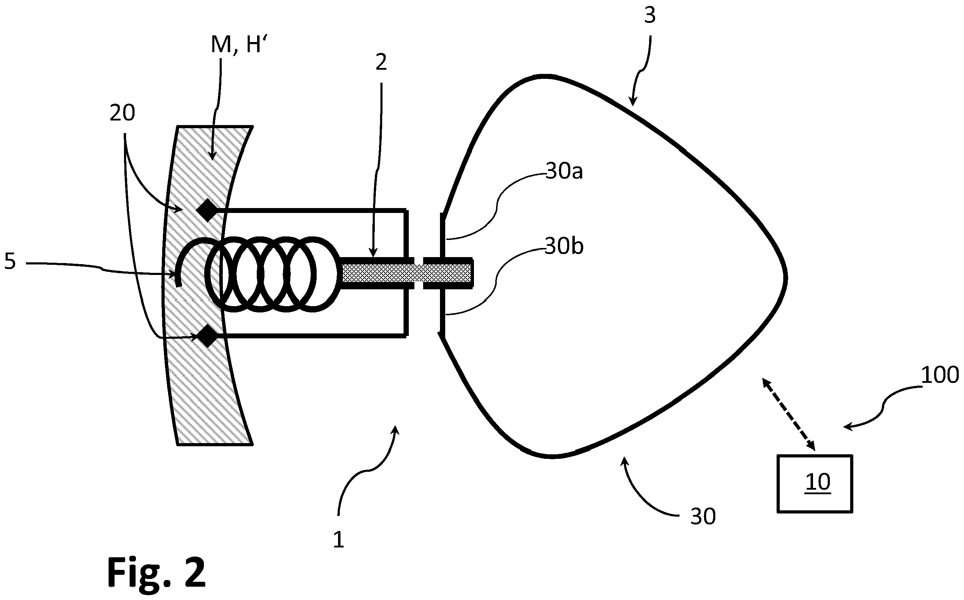

Gemäß einer ersten Variante, die z. B. in den Figuren 1 und 2 dargestellt ist, weist der Sensor 1 einen implantierbaren Körper mit einer Antennenstruktur 3, einer dielektrischen Komponente 2 und z. B. zwei Kontaktstellen 20 zum Gewebe M auf. Der Sensor 1 kann ein festes Gehäuse zur Aufnahme der Komponenten des Sensors aufweisen, dies ist jedoch nicht zwingend notwendig. So kann z. B. eine flexible Kunststofffolie als Trägermaterial (z. B. eine flexible Leiterbahn) verwendet werden. Je nach Material des Dielektrikums (Biokompatibilität) kann eine Einkapselung des Kondensators vorgesehen sein.

Durch Anlegen eines elektrischen Feldes (Reizüberleitung der Herzmuskelzellen) wird die Kapazität der dielektrischen Komponente 2 verändert. Dadurch ändert sich die Anpassung der Antennenstruktur 3, was mit Hilfe eines breitbandigen elektromagnetischen Signals, welches von einem implantierbaren medizinischen Gerät 10 (z. B. ICD oder Herzschrittmacher) zum Sensor 1 gesendet wird, gemessen werden kann.

Gemäß Fig. 1 kann die Antennenstruktur 3 zwei schlaufenförmige elektrische Leiter 30, 31 aufweisen, die jeweils über ihre Enden 30a, 30b bzw. 31a, 31b mit der dielektrischen Komponente 2 verbunden sind.

Der Sensor 1 kann weiterhin mittels eines Befestigungselements 5, hier z. B. in Form einer Schraube, mit dem Muskelgewebe M verbunden sein.

Figur 2 zeigt eine Abwandlung der in der Figur 1 gezeigten Ausführungsform, bei der die Antennenstruktur 3 durch einen einzelnen schlaufenförmigen elektrischen Leiter 30 gebildet ist, der über seine Enden 30a, 30b mit der dielektrischen Komponente 2 verbunden ist.

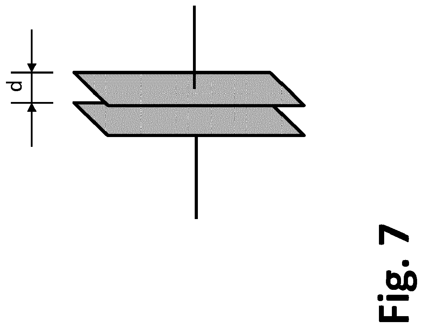

Ein grundlegendes Funktionsprinzip eines Sensors 1 gemäß Figuren 1 und 2 lässt sich wie folgt beschreiben. Ist die Änderung der relativen Permittivität bekannt, lässt sich die Änderung der Kapazität und damit die Verstimmung der Resonanzfrequenz der einfachen Kondensatoranordnung nach Figur 7 abschätzen. Aus der Publikation Rehrig, P., et. al., "Dielectric and Electromechanical Properties of Barium Titanate Single Crystals Grown by Templated Grain Growth", IEEE Transactions on Ultrasonics, Ferroelectrics, and Frequency Control, Vol. 47, No. 4, July 2000 kann z. B. die relative Permittivität als Funktion der Intensität des elektrischen Feldes für einen BaTiO₃-Kristall verwendet werden, um die relative Permittivität um den Nullpunkt herum annähernd zu εᵣ = 600-500*10⁻⁶*U/d zu errechnen.

Wird diese Abschätzung in die Gleichung zur Berechnung des Plattenkondensators C = ε₀*εᵣ*A/d nach Figur 7 eingesetzt, ergibt sich eine von der Spannung abhängige Kapazität (mit: C = Kapazität der Anordnung, ε₀ = Permittivität des Vakuums, A = Oberfläche der Kondensatorplatten, d = Abstand der Kondensatorplatten).

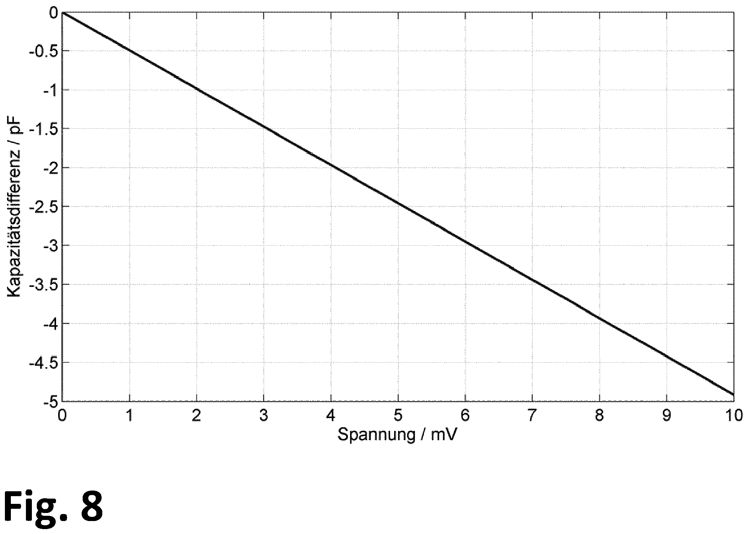

Werden beispielhaft Zahlenwerte eingesetzt (A = 1000 µm * 1000 µm, d = 3 µm), ergibt sich eine Änderung der Kapazität entsprechend Figur 8.

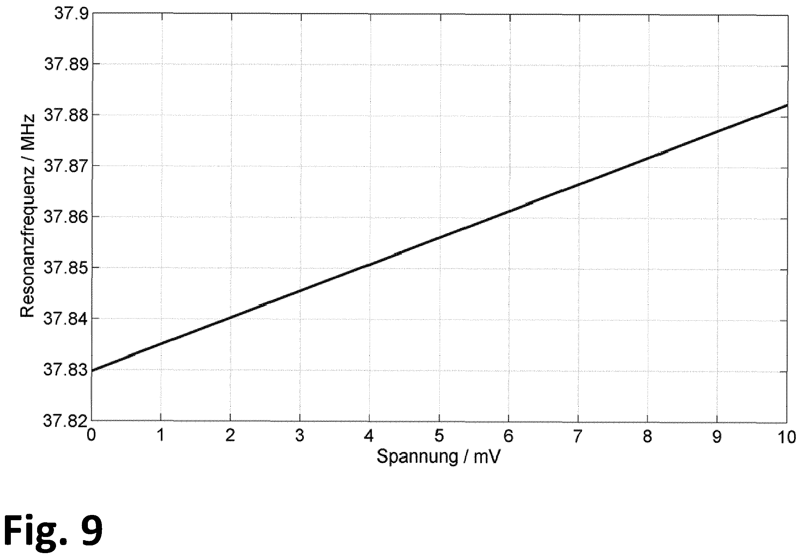

Mit der Thomsonschen Schwingungsgleichung f₀ = 1/(2*π*sqrt(L*C)) lässt sich durch einsetzen von L und C die Resonanzfrequenz eines Schwingkreises mit spannungsabhängiger Kapazität bestimmen. Werden die Parameter entsprechend von Figur 7 und Figur 8 gewählt und L = 10 nF gesetzt, ergibt sich eine Änderung der Resonanzfrequenz nach Figur 9, die in bekannter Weise messbar ist (z. B. durch das implantierbare medizinische Gerät 10).

Die jeweilige Antennenstruktur 3 (z. B. gemäß Figur 1 und 2) kann z. B. durch eine metallisch beschichteten Folie gebildet sein, wobei das Metall vorzugsweise Gold oder Platin ist, und wird vorzugsweise mittels des seriell geschalteten Dielektrikums 2 derart abgestimmt, dass die Kombination aus Antennenstruktur 3 und Kapazität 2 für ein Frequenzband im Ultrahochfrequenz-Bereich ohne ein äußeres statisches elektrisches Feld elektromagnetische Resonanz aufweist.

Das Dielektrikum 2 hat vorzugsweise im Falle eines äußeren angelegten elektrischen Feldes ein stark nichtlineares Verhalten und verstimmt somit je nach Feldstärke (Reizüberleitung der Herzmuskelzellen M) das Resonanzverhalten der Antennenstruktur 3. Geeignet dafür sind z. B. Keramikkondensatoren 2 der Klasse 2, insbesondere die Klasse-2-Keramiksorten Y5V und Z5U (nach EIA RS-198 bzw. nach IEC 60384-9/ IEC 60384-22: 2E6 und 2F4). Gemeinsam haben diese Keramiken, dass sie auf Basis ferroelektrischer Stoffe wie Bariumtitanat oder Blei-Zirkonat-Titanat gefertigt sind. Aufgrund seiner Biokompatibilität wird Bariumtitanat für Implantate bevorzugt.

Elektrische Signale, die durch Reizüberleitung bei Aktivität der Herzmuskelzellen entstehen, bilden schwache elektrische Felder aus, die eine direkte Auswirkung auf das Resonanzverhalten der Antennenstruktur 3 haben. Wird die Antennenstruktur 3 über einen Sender mit der Frequenz f0 im UHF-Bereich beispielsweise mit Hilfe von einem breitbandigen Impuls der Bandbreite Δf angeregt, wobei Δf im Bereich von 10 kHz bis 1 MHz liegt, so liefert das Reflexionsverhalten der Antennenstruktur 3 ein Maß für die Aktivität der Herzmuskelzellen M, die am Ort des Dielektrikums 2 herrscht.

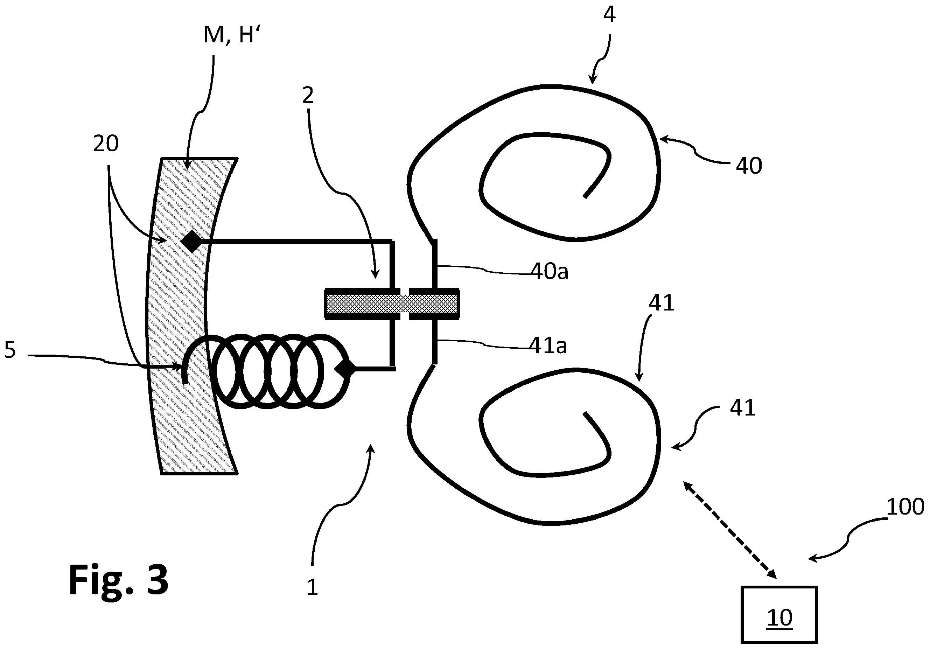

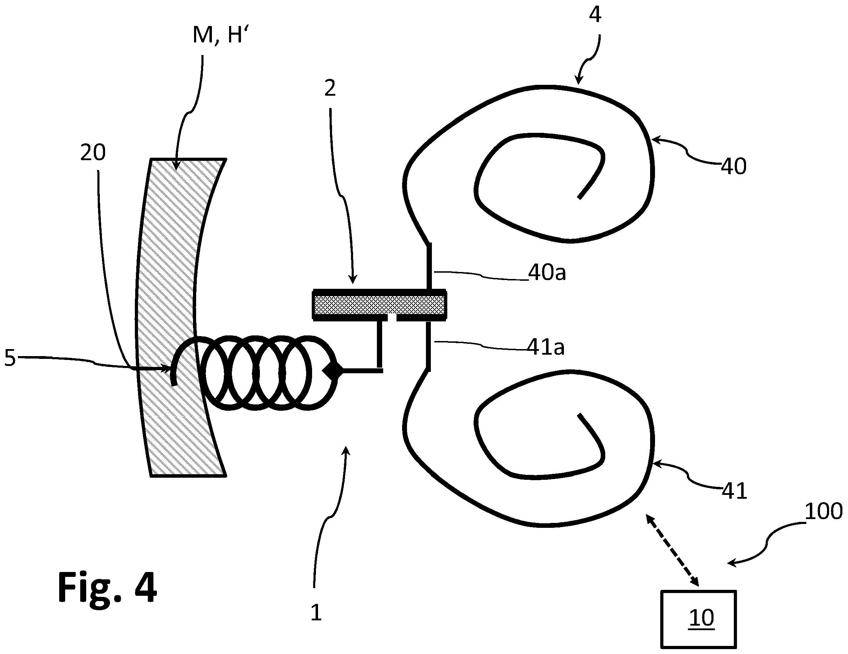

Die Figuren 3 und 4 zeigen jeweils eine alternative (zweite) Variante eines Sensors 1. Gemäß Figur 3 ist dabei z. B. vorgesehen, dass der Sensor 1 einen implantierbaren Körper mit einer Spulenstruktur 4, einer dielektrischen Komponente 2 und zumindest einer Kontaktstelle 20 zum Gewebe bzw. den Muskelzellen (hier insbesondere Herzmuskelzellen) M aufweist.

Das Dielektrikum 2 weist vorzugsweise wiederum im Falle eines äußeren angelegten elektrischen Feldes ein stark nichtlineares Verhalten auf und verstimmt somit je nach Feldstärke die Resonanzfrequenz des Schwingkreises 2, 4, da sich die Kapazität der dielektrischen Komponente 2 verändert. Die Änderung der Resonanzfrequenz des Schwingkreises aus Spulenstruktur 4 und dielektrischer Komponente 2, kann mit Hilfe der Nahfeldkopplung durch ein magnetisches Feld mit einem implantierbaren medizinischen Gerät 10 gemessen werden. Der Schwingkreis 2, 4 ist insbesondere vorzugsweise in einem Frequenzbereich resonant, in dem von einer rein magnetischen Kopplung ausgegangen werden kann.

Die Resonanzfrequenz ist weiterhin bevorzugt möglichst hoch gewählt, um einen kleinen Formfaktor und eine hohe Empfindlichkeit bei Änderung der Kapazität zu erhalten. Die Frequenz ist nach oben durch die Einhaltung der magnetischen Kopplung begrenzt.

Die Resonanzfrequenz kann insbesondere mit Hilfe aus der Literatur bekannter Verfahren durch das implantierbare medizinische Gerät 10 bestimmt werden und liefert somit ein Maß für die Aktivität der Herzmuskelzellen, die am Ort des Dielektrikums 2 herrscht.

Gemäß Figur 3 kann z. B. der Sensor 1 mittels eines Befestigungselements 5 (z. B. in Form einer Schraube) an dem Muskelgewebe M festgelegt sein, das zugleich als Kontaktstelle 20 fungiert. Der Sensor 1 bzw. die dielektrische Komponente 2 kann über eine weitere Kontaktstelle 20 mit den Muskelzellen M verbunden sein.

Alternativ hierzu kann der Sensor 2 lediglich über das Befestigungselement 5, das zugleich die Kontaktstelle 20 darstellt, mit den Muskelzellen bzw. Herzmuskelzellen M verbunden sein.

Die Spulenstruktur 4 der in den Figuren 3 und 4 gezeigten Ausführungsformen kann z. B. durch zwei spiralförmige elektrische Leiter 40, 41 gebildet sein, wobei die beiden spiralförmigen elektrischen Leiter 40, 41 jeweils über ein Ende 40a, 41a mit der dielektrischen Komponente 2 verbunden sind.

Die dielektrische Komponente 2 der Sensoren gemäß Figuren 3 und 4 basiert auch hier vorzugsweise auf einer ferroelektrischen Keramik (siehe oben) und kann z. B. ein Keramikkondensator der Klasse 2 sein, der wie oben beschrieben ausgebildet sein kann. Der Kondensator 2 kann in allen Ausführungsformen aus mehreren Keramikschichten bestehen (sogenannter multilayer ceramic capacitor MLCC).

Der Sensor 1, wie er z. B. in den Figuren 1 bis 4 in einzelnen Ausführungsformen gezeigt ist, ist weiterhin vorzugsweise jeweils zur Anordnung im Atrium A bzw. zur Implantation im Koronarsinus des Herzens H eingerichtet (als mögliche Position kommt auch das HIS-Bündel im Ventrikel in Frage) und vorgesehen, wobei die mindestens eine Kontaktstelle 20 (oder die beiden Kontaktstellen 20) vorzugsweise Muskelzellen bzw. Herzmuskelzellen M kontaktiert. Weiterhin ist der Sensor 1 vorzugsweise über das Befestigungselement 5 in der Herzwand H' verankert.

Des Weiteren kann ein Sensor 1 (vgl. z. B. Figuren 1 bis 4) zusammen mit einem implantierbaren medizinischen Gerät 10 ein System 100 bilden, wobei das Gerät 100 (z. B. ICD oder Herzschrittmacher) dazu ausgebildet ist, die Änderung der Anpassung der Antennenstruktur 3 (Figuren 1 und 2) bzw. die Änderung der Resonanzfrequenz des Schwingkreises 2, 4 (Figuren 3 und 4) zu messen, um die elektrische Erregung der Muskelzellen M drahtlos zu erfassen.

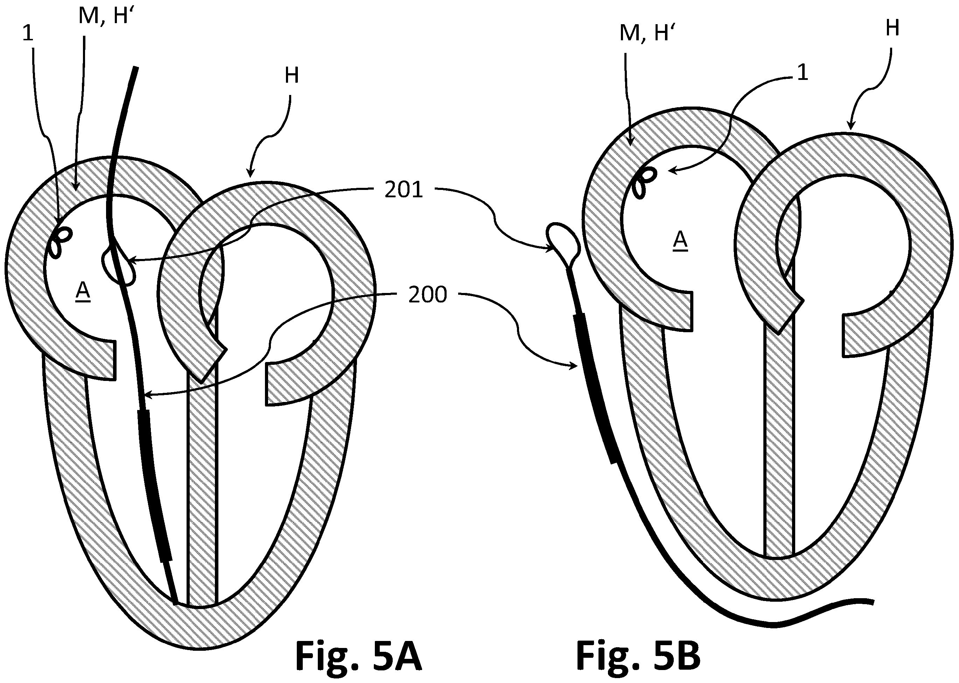

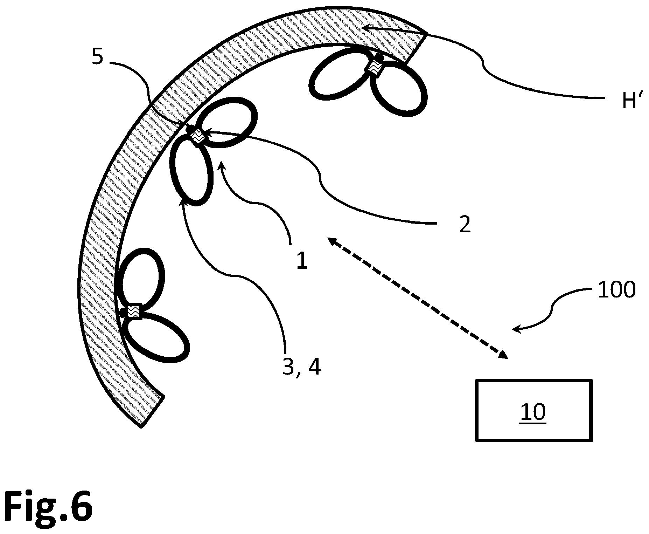

Die hier offenbarte Lösung verzichtet insbesondere mit Vorteil sowohl auf eine kabelgebundene Verbindung zum Sensor wie auch auf eine Batterie im Sensor 1 selber. Die Aktivität der Herzmuskelzellen M wird insbesondere drahtlos zwischen einem implantierbaren medizinischen Gerät 10 und dem Sensor 1 übertragen. Dadurch minimieren sich Kosten und Komplexität des Sensors 1. Durch den Verzicht auf eine zusätzliche kabelgebundene Verbindung zum Sensor ist das System 100 robust und erlaubt neue Ansätze bei der Therapie, wie zum Beispiel bei der extra-kardialen Brady-/ Tachykardietherapie (vgl. Fig. 5B). In den Figuren 6A und 6B bildet die Elektrodenleitung 200 sowohl einen Träger der Antenne 201 zur Abfrage der Signale des Sensors 1 als auch eine elektrische Verbindung zum aktiven Implantat.

Neben dem permanenten Messen der elektrischen Erregung der Muskelzellen M ist auch eine sequenzielle Abfrage der Messdaten (z. B. bei Bedarf zusätzlich zur herkömmlichen Messung oder zu Synchronisationszwecken) denkbar. Der kleine Formfaktor macht zudem die Implantation mehrerer der kabellosen Sensoren 1 an verschiedenen Positionen in der Herzwand H' möglich, wie es z. B. in der Figur 6 gezeigt ist. Die einzelnen Sensoren 1 können insbesondere verschiedene Resonanzfrequenzen aufweisen und können durch geeignete Pulse gezielt oder gemeinsam angesprochen werden.

## Patentansprüche

1. Implantierbarer Sensor (1) zur Detektion einer elektrischen Erregung von Muskelzellen (M), insbesondere Herzmuskelzellen (M), wobei der Sensor (1) eine dielektrische Komponente (2) sowie eine Kontaktstelle (20) zum Kontaktieren von Muskelzellen (M) aufweist, die mit der dielektrischen Komponente (2) verbunden ist, so dass sich bei einer elektrischen Erregung der Muskelzellen (M) ein elektrisches Feld in der dielektrischen Komponente (2) und entsprechend eine Kapazität der dielektrischen Komponente (2) ändert.

2. Implantierbarer Sensor nach Anspruch 1, wobei der Sensor (1) ein passiver Sensor (1) ist.

3. Implantierbarer Sensor nach Anspruch 1 oder 2, wobei der Sensor (1) zur drahtlosen Übertragung der Detektion einer Erregung der Muskelzellen (M) auf ein medizinisches Gerät (10), insbesondere ein implantierbares medizinisches Gerät (10), ausgebildet ist.

4. Implantierbarer Sensor nach einem der vorhergehenden Ansprüche, wobei der Sensor (1) eine Antennenstruktur (3) aufweist, die mit der dielektrischen Komponente (2) verbunden ist.

5. Implantierbarer Sensor nach Anspruch 4, wobei die Antennenstruktur (3) so mit der dielektrischen Komponente (2) verbunden ist, dass sich die Anpassung der Antennenstruktur (3) verändert, wenn sich bei einer elektrischen Erregung der Muskelzellen (M) das elektrische Feld in der dielektrischen Komponente (2) und die Kapazität der dielektrischen Komponente (2) ändern.

6. Implantierbarer Sensor nach Anspruch 4 oder 5, wobei die Antennenstruktur (3) einen elektrischen Leiter (30) aufweist, der ein erstes Ende (30a) und ein zweites Ende (30b) aufweist, wobei die beiden Enden (30a, 30b) jeweils mit der dielektrischen Komponente (2) verbunden sind.

7. Implantierbarer Sensor nach Anspruch 6, wobei der elektrische Leiter (30) schlaufenförmig ausgebildet ist.

8. Implantierbarer Sensor zur nach Anspruch 6 oder 7, wobei die Antennenstruktur (3) einen weiteren elektrischen Leiter (31) aufweist, der insbesondere schlaufenförmig ausgebildet ist, wobei der weitere elektrische Leiter (31) ein erstes Ende (31a) und ein zweites Ende (31b) aufweist, wobei die beiden Enden (31a, 31b) des weiteren elektrischen Leiters (31) jeweils mit der dielektrischen Komponente (2) verbunden sind.

9. Implantierbarer Sensor nach einem der Ansprüche 1 bis 3, wobei der Sensor (1) eine Induktivität in Form einer Spulenstruktur (4) aufweist, die mit der dielektrischen Komponente (2) verbunden ist.

10. Implantierbarer Sensor nach Anspruch 9, wobei die Spulenstruktur (4) und die dielektrische Komponente (2) einen Schwingkreis bilden, so dass sich eine Resonanzfrequenz des Schwingkreises ändert, wenn sich bei einer elektrischen Erregung der Muskelzellen (M) das elektrische Feld in der dielektrischen Komponente (2) und die Kapazität der dielektrischen Komponente (2) ändern.

11. Implantierbarer Sensor nach Anspruch 9 oder 10, wobei die Spulenstruktur (4) zwei spiralförmige elektrische Leiter (40, 41) aufweist, wobei die beiden spiralförmigen elektrischen Leiter (40, 41) jeweils mit der dielektrischen Komponente (2) verbunden sind, wobei insbesondere der jeweilige spiralförmige elektrische Leiter (40, 41) über ein Ende (40a, 41a) des jeweiligen spiralförmigen Leiters (40, 41) mit der dielektrischen Komponente (2) verbunden ist.

12. Implantierbarer Sensor nach einem der vorhergehenden Ansprüche, wobei der Sensor (1) ein Befestigungselement (5) zum Befestigen des Sensors (1) am Gewebe eines Patienten aufweist, wobei das Befestigungselement (5) insbesondere an der dielektrischen Komponente (2) festgelegt ist.

13. Implantierbarer Sensor nach Anspruch 12, wobei die Kontaktstelle (20) durch das Befestigungselement (5) gebildet ist.

14. Implantierbarer Sensor nach einem der vorhergehenden Ansprüche, wobei der Sensor (1) eine weitere Kontaktstelle (20) zum Kontaktieren von Muskelzellen (M) aufweist, die mit der dielektrischen Komponente (2) verbunden ist.

15. System (100), aufweisend zumindest einen implantierbaren Sensor (1) nach einem der vorhergehenden Ansprüche sowie ein implantierbares medizinisches Gerät (10), wobei das medizinische Gerät (10) zum Messen einer von dem mindestens einen Sensor (1) detektierten Erregung von Muskelzellen (M) konfiguriert ist.
